# EUROPEAN PATENT SPECIFICATION

(11) **EP 0 473 993 B1**
(45) Date of publication and mention of the grant of the patent: **17.04.1996**
(21) Application number: 91113707.3
(22) Date of filing: 15.08.1991
(51) Int. Cl.: C07K 5/08, C07K 5/06, A61K 38/04

(54) **Novel peptide derivatives and their pharmaceutical use**
Neue Peptidderivate und deren pharmazeutischer Gebrauch
Nouveaux dérivés peptidiques et leur emploi pharmaceutique

(30) Priority: 17.08.1990 JP 215752/90
(43) Date of publication of application: 11.03.1992
(73) Proprietor: Japan Tobacco Inc., Shinagawa-ku, Tokyo 140 (JP)
(72) Inventor: Haruta, Junichi, c/o Pharmaceutical Research, Midori-ku, Yokohama-shi, Kanagawa 227 (JP); Yasuda, Akihiro, c/o Pharmaceutical Research, Midori-ku, Yokohama-shi, Kanagawa 227 (JP); Hara, Katsuyoshi, c/o Pharmaceutical Research, Midori-ku, Yokohama-shi, Kanagawa 227 (JP); Iwata, Kunio, c/o Toxicology Research, Hatano-shi, Kanagawa 257 (JP); Furukawa, Noboru, c/o Toxicology Research, Hatano-shi, Kanagawa 257 (JP); Uchida, Itsuo, c/o Pharmaceutical Research, Midori-ku, Yokohama-shi, Kanagawa 227 (JP)
(74) Representative: von Kreisler, Alek, Dipl.-Chem.

(56) References cited:
- EP-A- 0 144 230
- EP-A- 0 171 159
- FR-A- 2 460 291
- CHEMICAL ABSTRACTS, vol. 92, no. 5, 4 February 1980, Columbus, Ohio, US; abstract no. 42368f, L G BAUCE ET AL. 'synthesis of a series of residue 1 (pyroglutamic acid) analogs of thyrotropin releasing hormone' page 825 ;column LEFT ;

## Description

### BACKGROUND OF THE INVENTION

This invention relates to peptide derivatives of the following formula [I] and pharmaceutically acceptable acid addition salts thereof which exhibit more potent stimulant activities in central nerve system such as an antagonistic action against hypothermia, a locomoter stimulant action, a spinal reflex stimulant action, etc., as compared with other pharmaceuticals, particularly thyrotropin releasing hormone (TRH) and its derivatives, and thus are useful as a therapeutic medicament for central nervous disorders: 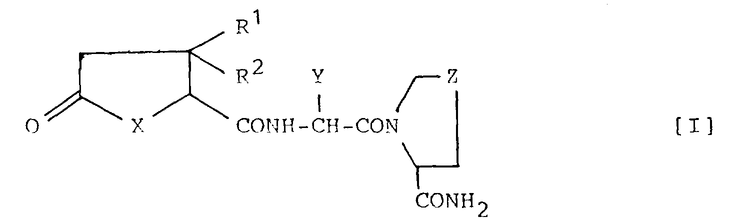 wherein R¹ and R are the same or different and respectively mean a hydrogen atom, a C₁₋₅ alkyl group or a phenyl group, X is -NH- or -CH₂-, Y is a hydrogen atom, a benzyl group wherein phenyl may be substituted by hydroxyl, or a C₁₋₅ alkyl group which may be substituted by -SH, -SR³, -SO₂R³, -CONH₂, 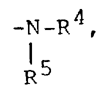 -OR⁶ or 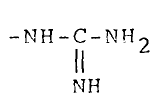 (wherein R³ means a C₁₋₅ alkyl group or an aryl group, R⁴ and R⁵ are the same or different and respectively mean a hydrogen atom, a C₁₋₅ alkyl group or an amino protecting group and R⁶ is a hydrogen atom, a C₁₋₅ alkyl group or a hydroxyl protecting group), and Z is -CH₂- or -S-, provided that when X is -NH-, R¹ and R are not hydrogen atoms at the same time.

TRH is a tripeptide (L-pyroglutamyl-L-histidyl-L-prolinamide; pGlu-His-Pro-NH₂) synthesized in cerebral hypothalamus. TRH is known to have thyroid stimulating hormone (TSH)-releasing activity and is also known to be useful as a therapeutic medicine for disturbance of consciousness caused by cerebral function disturbances. However, TRH has defects such that it is poorly stable in vivo, easily decomposed by pyroglutamylpeptidase, TRH amidase and other enzymes [Progress of Medicines (Igaku no Ayumi), vol. 134, No. 4, p. 252] and deactivated in a short period, necessitating frequent administration when used, which results in excessive secretion of TSH. For the improvement of the above-mentioned defects of TRH, studies from various aspects have been conducted for TRH derivatives which on the one hand reduce TSH secretion-inducing action as the side effect and on the other hand possess more potent central nerve-stimulant actions such as an antihypnotic action, an anti-reserpine action (antagonistic action against hypothermia), a locomoter stimulant action, a spinal reflex stimulant action, a dopamine potentiating action and an anesthesia antagonism, and have more excellent durability in action than TRH. For example, there have been knowm TRH derivatives in which the pGlu-moiety of TRH is converted into a heterocyclic group such as 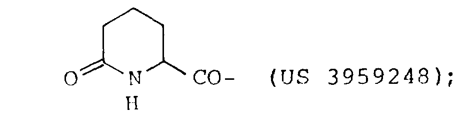 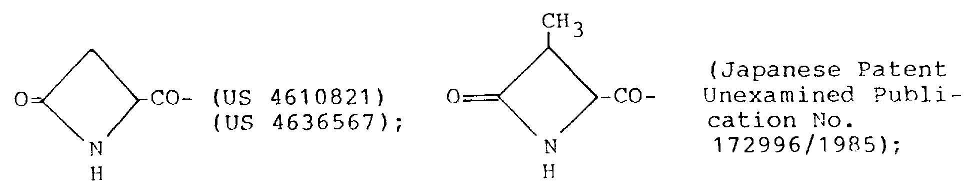 or the like. Also, TRH derivatives wherein the His moiety is converted to leucine (Leu) have been reported in US 4299821 and others.

Inventors of the present invention previously invented TRH derivatives wherein the pGlu-moiety was converted. As a result of further intensive studies on TRH derivatives, the inventors have found that the TRH derivatives obtained by converting the pGlu-L-His-moiety overcome the defects of TRH, and possess more potent stimulant actions on central nervous system and longer durability than other known TRH derivatives, which resulted in completion of the invention.

### SUMMARY OF THE INVENTION

The object of the present invention is to provide novel peptide derivatives and pharmaceutically acceptable acid addtion salts thereof which are obtained by converting the pGlu-L-His-moiety of TRH into a structure of the following formula [II] 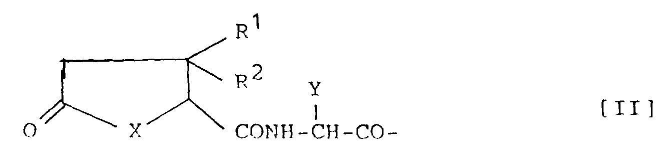 wherein n, R¹, R, X and Y are as defined above, and have marked characteristic feature in terms of chemical structure.

According to the present invention, the novel peptide derivatives and pharmaceutically acceptable acid addition salts thereof are represented by the following formula [I]: 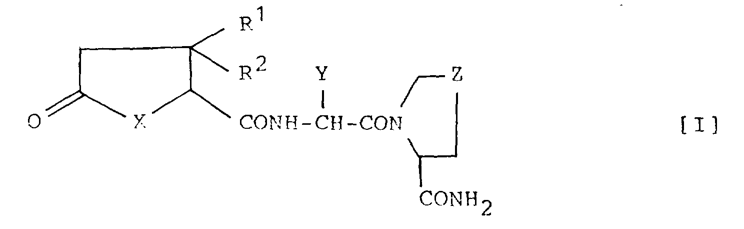 wherein R¹ and R are the same or different and respectively mean a hydrogen atom, a C₁₋₅ alkyl group or a phenyl group, X is -NH- or -CH₂-, Y is a hydrogen atom, a benzyl group wherein phenyl may be substituted by hydroxyl, or a C₁₋₅ alkyl group which may be substituted by -SH, -SR³, -SO₂R³, -CONH₂, 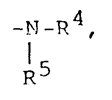 -OR⁶ or 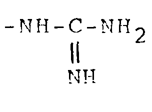 (wherein R³ means a C₁₋₅ alkyl group or an aryl group, R⁴ and R⁵ are the same or different and respectively mean a hydrogen atom, a C₁₋₅ alkyl group or an amino protecting group and R⁶ is a hydrogen atom, a C₁₋₅ alkyl group or a hydroxyl protecting group), and Z is -CH₂- or -S-, provided that when X is -NH-, R¹ and R are not hydrogen atoms at the same time.

### DETAILED DESCRIPTION OF THE INVENTION

In the present specification, the C₁₋₅ alkyl group means a straight or branched carbon-chain having, preferably, one through four carbon atoms, which is exemplified by methyl group, ethyl group, propyl group, butyl group, isopropyl group, and sec-butyl group. The aryl group means, for example, phenyl, and tolyl, the amino protecting group means, for example, acetyl, benzoyl, benzyl, tosyl, benzyloxycarbonyl, tert-butoxycarbonyl, and 2,4-dinitrophenyl, and the hydroxyl protecting group means, for example, benzyl, acetyl, and benzoyl.

The objective compounds [I] of this invention are capable of forming pharmaceutically acceptable salts thereof with an acid. Examples of the acid include inorganic acids such as hydrochloric acid and sulfuric acid or organic acids such as citric acid, acetic acid and tartaric acid. These salts are also encompassed in the present invention.

The objective compounds [I] of the present invention have at least three asymmetric carbons, and there exist stereoisomers based on such asymmetric carbons. The objective compounds of the present invention include respective isolated stereoisomers and the mixtures of the stereoisomers.

According to the present invention, the objective compounds [I] or their acid addition salts can be produced by the following methods:
(A) a condensing compound of the formula [III] 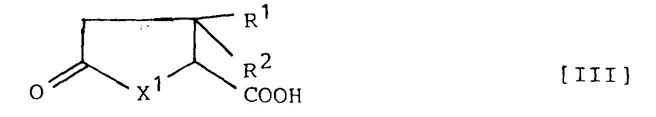 wherein R¹ and R are as defined above and X¹ is imino group, a protected imino group or methylene group, or its reactive derivative with a compound of the formula [IV] 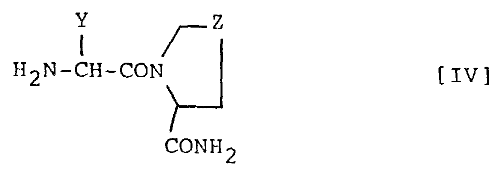 wherein Y and Z are as defined above, or its salt;
(B) condensing a compound of the formula [V] 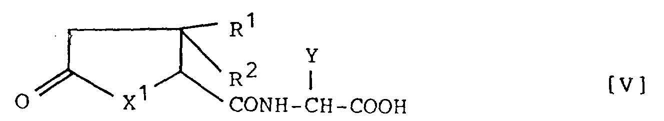 wherein R¹, R, X¹ and Y are as defined above, its salt or its reactive derivative with an amino acid amide of the formula [VI] 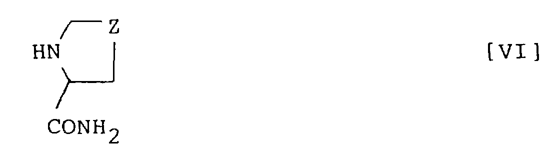 wherein Z is as defined above, or its salt; or
(C) subjecting a compound of the formula [VII] 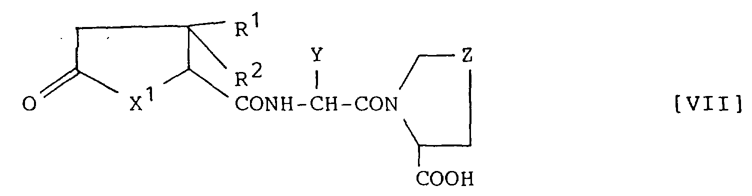 wherein R¹, R, X¹, Y and Z are as defined above, its salt or its reactive derivative to amidation, and
(D) thereafter, when X¹ in the product thus obtained by the above step (A), (B) or (C) is a protected imino group, removing the protective group ; and
(E) whereafter if desired, converting the thus-obtained product into an acid addition salt thereof, to yield the objective compound.

The starting compounds [IV] - [VII] of the present invention can be subjected to the respective reaction either in the form of free base or in the form of salt. The salt can be preferably used in the form of acid addition salt, and as such acid addition salt, use can be made of, for example, an inorganic acid addition salt such as hydrochloride or hydrobromide, or an organic acid addition salt such as trifluoroacetate or p-toluenesulfonate.

As the reactive derivative of the compounds [III], [V] and [VII], use can be made of an active ester (e.g. N-hydroxysuccinimido ester, pentachlorophenyl ester, N-hydroxybenzotriazole ester), an acid halide (e.g. acid chloride), an acid azide, an acid anhydride and an imidazolamide, corresponding to the respective compounds. The active ester may be subjected to peptide-synthesizing reaction after isolated or without being isolated. As the reactive derivative of the compound [VII], use can be preferably made of, for example, an ester (an alkyl ester such as ethyl ester or methyl ester; or an aralkyl ester such as benzyl ester).

When the group X¹ in the above-mentioned starting compounds [III], [V] and [VII] is a protected imino group, use can be made of any protective group conventionally used in peptide synthesis as the protective group of the imino group. Examples of the suitable protective group include benzyloxycarbonyl group, benzyl group, tosyl group, tert-butoxycarbonyl group and 2,4-dinitrophenyl group.

### [Reaction Steps (A) and (B)]

Both of the condensation reaction of a compound [III] or a reactive derivative thereof with a compound [IV] or a salt thereof and the condensation reaction of a compound [V] or a salt thereof or a reactive derivative thereof with a compound [VI] or a salt thereof, which are peptide synthetic reactions, can be carried out by a conventional means. For example, the condensation reaction of a compound [III] with a compound [IV] or a salt thereof and that of a compound [V] or a salt thereof with a compound [VI] or a salt thereof can be carried out in a suitable solvent in the presence of a condensing agent. Examples of the suitable condensing agent include dicyclohexylcarbodiimide, 1-ethyl-3-(3-dimethylaminopropyl)carbodiimide, phosphorus oxychloride, phosphorus trichloride, thionyl chloride, oxalyl chloride, triphenylphosphine and Vilsmeier Reagent. The condensation reaction is carried out preferably at -50 - 50°C. When a salt of a compound [IV] is used, the reaction is conducted in the presence of a deacidifying agent.

On the other hand, the condensation reaction of a reactive derivative of a compound [III] with a compound [IV] or a salt thereof and that of a reactive derivative of a compound [V] with a compound [VI] or a salt thereof can be carried out in a suitable solvent in the presence or absence of a deacidifying agent. As the deacidifying agent, mention can be made of, for example, trialkylamines (triethylamine and trimethylamine), N,N-dialkylanilines (N,N-dimethylaniline and, N,N-diethylaniline), pyridine, N-alkylmorpholines (N-methylmorpholine), alkali metal hydroxides (potassium hydroxide and sodium hydroxide), alkali metal carbonates (potassium carbonate) and alkali metal hydrogencarbonates (sodium hydrogencarbonate). This condensation reaction can be suitably carried out at -50 - 50°C, particularly -10 - 10°C.

As the solvent for the condensation reaction usable in the reaction steps (A) and (B), use can be made of, for example, N,N-dimethylformamide, dichloromethane, dioxane, tetrahydrofuran, acetonitrile, ethyl acetate, pyridine, acetone and water.

### [Reaction Step (C)]

The amidation reaction of a compound [VII], a salt thereof or a reactive derivative thereof can be carried out by reacting the compound with ammonia or an ammonia-donating compound. For example, the amidation reaction of a compound [VII] or a salt thereof with ammonia or an ammonia-donating compound can be preferably carried out in the presence of a dehydrating agent in a suitable solvent. As the dehydrating agent, use can be made of, for example, any compound mentioned above as the suitable condensing agent. As the ammonia-donating compound, use can be made of any compound capable of releasing ammonia in the reaction mixture, which is exemplified by ammonium chloride and ammonium carbonate. The amidation reaction is preferably carried out at -20°C - 20°C. As the suitable solvent, mention can be made of, for example, N,N-dimethylformamide, dimethylsulfoxide and tetrahydrofuran. Furthermore, the amidation reaction of a reactive derivative of a compound [VII] or a salt thereof with ammonia or an ammonia-donating compound can be carried out in the presence or absence of a deacidifying agent in a suitable solvent. As the deacidifying agent, use can be made of any of the deacidifying agents mentioned in the description of the above reaction steps (A) and (B). The amidation reaction is preferably carried out at -20°C - 20°C. As the suitable solvent, mention can be made of methanol, ethanol, N,N-dimethylformamide and dimethylsulfoxide.

### [Reaction Step D]

When X¹ in the compound produced in the above-mentioned reaction step (A), (B) or (C) is a protected imino, the deprotection of the protective group can be carried out easily, depending upon the species of the protective group, in accordance with a conventional method such as catalytic reduction, electrolytic reduction, acid treatment, base treatment or oxidation reaction.

### [Reaction Step (E)]

The thus-obtained objective compound can be, if necessary, converted into an acid addition salt easily by treating with a stoichiometric amount of an acid in accordance with a conventional method.

In the above-mentioned reactions, the staring compounds [III] - [VII], the intermediate products and the objective compounds contain 1-4 asymmetric carbon atom(s), and the above methods of the present invention can be carried out using the respective optically active isomers of the starting compounds [III] - [VII] or their respective mixture. Since the above-mentioned reactions of the present invention can be carried out without involving racemization, the intermediate products and the objective compounds can be obtained in the form of the corresponding optically active isomer when the respective optically active isomers of the starting compounds [III] - [VII] are used.

The objective compounds [I] and pharmaceutically acceptable acid addition salts thereof can be administered orally or nonorally as they are or in the form of, for example, powders, granules, tablets, capsules, injections (intravenous, subcutaneous, intramuscular) or suppositories suitably in admixture with pharmacologically acceptable carriers, excipients and diluents.

While the dosage of the objective compounds [I] or their pharmaceutically acceptable acid addition salts of the present invention varies depending on the administration route, the age, body weight or symptom of the patient, generally it is preferably 0.5 µg - 5 mg/kg/day. Particularly, in the case of oral administration, the dosage is preferably 10 µg - 5 mg/kg/day and in the case of parenteral administration (e.g. intravenous administration, intramuscular administration, subcutaneous administration), the dosage is preferably 1 - 1000 µg/kg/day.

Since the compounds [I] or their pharmaceutically acceptable acid addition salts of the present invention possess more potent actions on central nervous system (e.g. antagonistic action against hypothermia, locomotor stimulant action, spinal reflex stimulant action), they are of use as a therapeutic medicament for central nervous disorders such as impaired consciousness, depression and hypomnesia, in association with schizophrenia, melancholia, senile dementia, sequelae of cerebrovascular disorders, head trauma, epilepsy, and spinocerebellar degeneracy.

Below, the present invention is in further detail described by illustrating reference examples and working examples. The abbreviations used in the examples stand for the following meanings respectively.
- NMR: nuclear magnetic resonance spectra (¹H-NMR)
- CIMS: chemical ionization mass spectra
- DMF: N,N-dimethylformamide
- HOBT: 1-hydroxy-1,2,3-benzotriazole
- DCC: 1,3-dicyclohexylcarbodiimide

Hydrobromide of dipeptide of the formula [IV] which is used in the present invention was synthesized by the method of T. Szirtes et al [T. Szirtes et al, J. Med. Chem., 27, 741 (1984)].

### Example 1

N-(cis-3-Methylpyroglutamyl)-L-leucyl-L-prolinamide (Compound 1A and 1B)

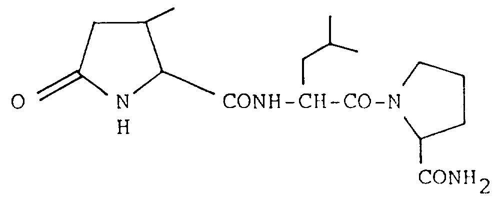

L-Leucyl-L-prolinamide hydrobromide (517 mg) was dissolved in DMF (1.5 ml), to which was added triethylamine (0.35 ml) under cooling at -10°C. The mixture was stirred for 10 minutes under ice-cooling and the resultant precipitate was filtered off to give L-leucyl-L-prolinamide in DMF solution, which was immediately used for the next reaction.

cis-3-Methylpyroglutamic acid [A. B. Mauger, J. Org. Chem., 46, 1032 (1981)] (200 mg) was dissolved in DMF (1.5 ml), to which was added HOBT (257 mg), followed by cooling to 0°C. DCC (317 mg) was added thereto and the mixture was stirred at 0°C overnight. To the mixture was added the above-mentioned L-leucyl-L-prolinamide in DMF, and the mixture was stirred at 5°C overnight. The resultant precipitate was filtered off and concentrated to dryness under reduced pressure. The residue was washed with water and purified by HP-20 column chromatography (1.0x40cm) with methanol as eluent. The eluted fraction was concentrated to dryness under reduced pressure, and the residue obtained was purified by silica gel column chromatography (developing solvent: chloroform:methanol:ammonia water=60:10:1) to give the title less polar compound 1A (188 mg) and the more polar compound 1B (177 mg) as powders. The compounds 1A and 1B have diastereomeric relationship to each other.
Compound 1A
   NMR (CD₃OD)
   δppm: 0.90 - 1.10 (9H, m), 1.55 - 1.82 (3H, m), 1.89 - 2.29 (5H, m), 2.35 - 2.44 (1H, m), 2.72 - 2.88 (1H, m), 3.61 - 3.71 (1H, m), 3.86 - 3.98 (1H, m), 4.19 (1H, d), 4.35 - 4.45 (1H, m), 4.59 - 4.68 (1H, m)
   CIMS m/z: 353(M+1)⁺
Compound 1B
   NMR (CD₃OD)
   δ ppm: 0.90 - 1.10 (9H, m), 1.55 - 1.84 (3H, m), 1.55 - 1.84 (3H, m), 1.85 - 2.29 (5H, m), 2.30 - 2.44 (1H, m), 2.72 - 2.90 (1H, m), 3.61 - 3.72 (1H, m), 3.85 - 3.95 (1H, m), 4.15 (1H, d), 4.43 - 4.53 (1H, m), 4.60 - 4.69 (1H, m)

### Example 2

N-(cis-3-Methylpyroglutamyl)-L-methionyl-L-prolinamide (Compound 2A and 2B)

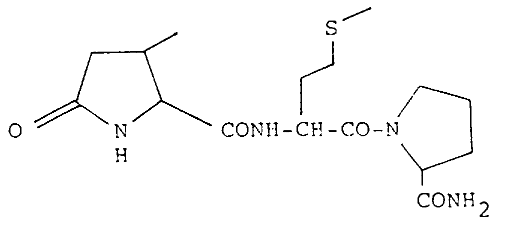

In the same manner as in Example 1, the title less polar compound 2A (140 mg) and the more polar compound 2B (150 mg) were obtained each as a powder from L-methionyl-L-prolinamide hydrobromide (685 mg) and cis-3-methylpyroglutamic acid (300 mg). The compounds 2A and 2B have diastereomeric relationship to each other.
Compound 2A
   NMR (CD₃OD)
   δ ppm: 1.00 (3H, d, J=7.0Hz), 1.86 - 2.30 (10H, m), 2.39 (1H, d, J=8.5Hz and 16.5Hz), 2.51 - 2.72 (2H, m), 2.73 - 2.88 (1H, m), 3.69 - 3.80 (1H, m), 3.90 - 3.99 (1H, m), 4.18 (1H, d, J=8.0Hz), 4.40 (1H, dd, J=4.8Hz and 8.3Hz)
   CIMS m/z: 371 (M+1)⁺
Compound 2B
   NMR (CD₃OD)
   δ ppm: 1.05 (3H, d, J=7.0Hz), 1.86 - 2.26 (10H, m), 2.37 (1H, d, J=8.5Hz and 16.5Hz), 2.52 - 2.72 (2H, m), 2.73 - 2.86 (1H, m), 3.68 - 3.70 (1H, m), 3.86 - 3.96 (1H, m), 4.15 (1H, d, J=8.1Hz), 4.41 (1H, dd, J=4.8Hz and 8.2Hz), 4.82 (1H, dd, J=5.2Hz and 8.9Hz)

### Example 3

N-(cis-3-Methylpyroglutamyl)-L-norvalyl-L-prolinamide (Compound 3A and 3B)

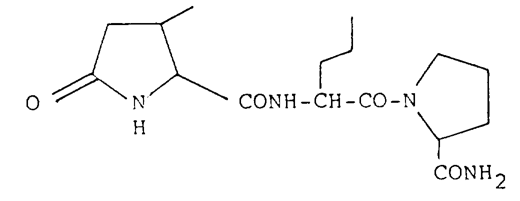

In the same manner as in Example 1, the title less polar compound 3A (250 mg) and the more polar compound 3B (241 mg) were obtained each as a powder from L-norvalyl-L-prolinamide hydrobromide (618 mg) and cis-3-methylpyroglutamic acid (300 mg). The compounds 3A and 3B have diastereomeric relationship to each other.
Compound 3A
   NMR (CD₃OD)
   δppm: 0.89 - 1.10 (6H, m), 1.35 - 1.55 (2H, m), 1.55 - 1.74
   (1H, m), 1.74 - 2.29 (6H, m), 2.38 (1H, dd, J=8.5Hz and 16.4Hz), 2.71 - 2.88 (1H, m), 3.60 - 3.72 (1H, m), 3.85 - 3.97 (1H, m), 4.19 (1H, d, J=8.0Hz), 4.39 (1H, dd, J=4.7Hz and 8.2Hz), 4.64 (1H, dd, J=5.2Hz and 8.9Hz)
   CIMS m/z: 339(M+1)⁺
Compound 3B
   NMR (CD₃OD)
   δppm: 0.97 (3H, t, J=7.3Hz), 1.05 (3H, d, J=7.0Hz), 1.35 -
   1.60 (2H, m), 1.60 - 1.73 (1H, m), 1.73 - 2.30 (6H, m), 2.35 (1H, dd, J=8.5Hz and 16.5Hz), 2.71 - 2.86 (1H, m), 3.61 - 3.72 (1H, m), 3.82 - 3.93 (1H, m), 4.16 (1H, d, J=8.1Hz), 4.39 (1H, dd, J=4.7Hz and 8.2Hz), 4.57 (1H, dd, J=4.9Hz and 9.1Hz)

### Example 4

### N-(cis-3-Methylpyroglutamyl)-L-phenylalanyl-L-prolinamide (Compound 4A and 4B)

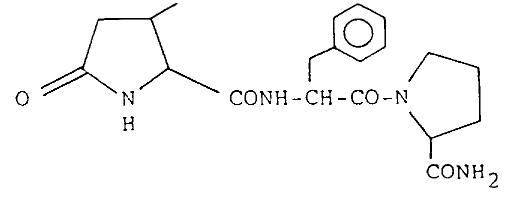

In the same manner as in Example 1, the title less polar compound 4A (250 mg) and the more polar compound 4B (150 mg) were obtained each as a powder from L-phenylalanyl-L-prolinamide hydrobromide (719 mg) and cis-3-methylpyroglutamic acid (300 mg). The compounds 4A and 4B have diastereomeric relationship to each other.
Compound 4A
   NMR (CD₃OD)
   δ ppm: 0.84 (2.4H, d, J=7.0Hz), 1.01 (0.6H, d, J=7.0Hz), 1.61 - 2.25 (5H, m), 2.33 (1H, dd, J=8.5Hz and 16.6Hz), 2.67 - 2.82 (1H, m), 2.86 - 3.20 (1H, m), 3.15 - 3.25 (1H, m), 3.47 - 3.68 (1H, m), 3.88 - 3.99 (1H, m), 4.11 (0.8H, d, J=8.1Hz), 4.15 (0.2H, d, J=8.0Hz), 4.35 - 4.46 (1H, m), 7.15 - 7.42 (5H, m)
   CIMS m/z: 387(M+1)⁺
Compound 4B
   NMR (CD₃OD)
   δ ppm: 0.57 (2.4H, d, J=7.0Hz), 0.94 (0.6H, d, J=7.0Hz), 1.62 - 2.41 (6H, m), 2.55 - 2.72 (1H, m), 2.81 - 3.03 (1H, m), 3.16 - 3.28 (1H, m), 3.58 - 3.77 (1H, m), 3.85 - 3.97 (1H, m), 4.08 (0.8H, d, J=8.0Hz), 4.16 (0.2H, d, J=8.0Hz), 4.35 - 4.45 (1H, m), 7.15 - 7.45 (5H, m)

### Example 5

N-(cis-3-Methylpyroglutamyl)-L-norleucyl-L-prolinamide (Compound 5A and 5B)

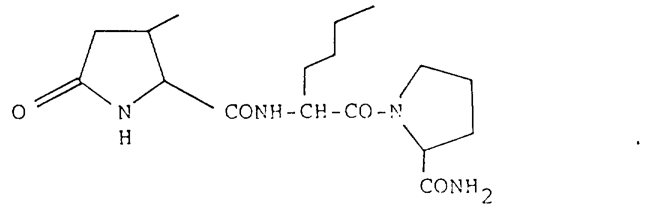

In the same manner as in Example 1, the title less polar compound 5A (220 mg) and the more polar compound 5B (160 mg) were obtained each as a powder from L-norleucyl-L-prolinamide hydrobromide (647 mg) and cis-3-methylpyroglutamic acid (300 mg). The compounds 5A and 5B have diastereomeric relationship to each other.
Compound 5A
   NMR (CD₃OD)
   δppm: 0.82 - 1.13 (6H, m), 1.25 - 1.55 (4H, m), 1.58- 1.77 (1H, m), 1.77 - 2.31 (6H, m), 2.38 (1H, dd, J=8.5Hz and 16.5Hz), 2.70 - 2.90 (1H, m), 3.57 - 3.72 (1H, m), 3.83 - 3.99 (1H, m), 4.19 (1H, d, J=8.0Hz), 4.39 (1H, dd, J=4.7Hz and 8.2Hz), 4.62 (1H, dd, J=5.1Hz and 9.0Hz)
   CIMS m/z: 353(M+1)⁺
Compound 5B
   NMR (CD₃OD)
   δppm: 0.94 (3H, t, J=6.9Hz and 7.1Hz), 1.05 (3H, d, J=7.0Hz), 1.29 - 1.55 (4H, m), 1.55 - 1.76 (1H, m), 1.76 - 2.31 (6H, m), 2.35 (1H, dd, J=8.5Hz and 16.5Hz), 2.69 - 2.89 (1H, m), 3.57 - 3.74 (1H, m), 3.80 - 3.96 (1H, m), 4.16 (1H, d, J=8.1Hz), 4.39 (1H, dd, J=4.7Hz and 8.2Hz), 4.56 (1H, dd, J=4.9Hz and 9.2Hz)

### Example 6

N-(3,3-Dimethylpyroglutamyl)-L-leucyl-L-prolinamide (Compound 6A and 6B)

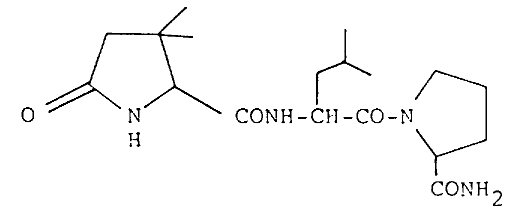

In the same manner as in Example 1, the title less polar compound 6A (85 mg) and the more polar compound 6B (63 mg) were obtained each as a powder from L-leucyl-L-prolinamide hydrobromide (432 mg) and 3,3-dimethylpyroglutamic acid [T. Yamazaki et al, Chem. Pharm. Bull., 24, 3011 (1976)] (200 mg). The compounds 6A and 6B have diastereomeric relationship to each other.
Compound 6A
   NMR (CD₃OD)
   δppm: 0.96 (3H, d, J=6.0Hz), 0.98 (3H, d, J=6.0Hz), 1.02 (3H, s), 1.26 (3H, s), 1.60 - 1.70 (2H, m), 1.70 - 1.80 (1H, m), 1.90 - 2.30 (4H, m), 2.04 (1H, d, J=16.5Hz), 2.30 (1H, d, J=16.5Hz), 3.60 - 3.70 (1H, m), 3.83 (1H, s), 3.87 - 3.98 (1H, m), 4.39 (1H, dd, J=8.0Hz and 4.5Hz), 4.7 (21H, dd, J=9.0Hz and 5.0Hz)
   CIMS m/z: 367(M+1)⁺
Compound 6B
   NMR (CD₃OD)
   δppm: 0.96 (3H, d, J=6.5Hz), 0.99 (3H, d, J=6.5Hz), 1.08 (3H, s), 1.26 (3H, s), 1.55 - 1.70 (2H, m), 1.70 - 1.85 (1H, m), 1.85 - 2.30 (4H, m), 2.02 (1H, d, J=16.5Hz), 2.36 (1H, d, J=16.5Hz), 3.60 - 3.70 (1H, m), 3.80 (1H, s), 3.83 - 3.95 (1H, m), 4.39 (1H, dd, J=8.0Hz and 5.5Hz), 4.63 (1H, dd, J=10.0Hz and 4.5Hz)

### Example 7

N-(3,3-Dimethylpyroglutamyl)-L-norvalyl-L-prolinamide (Compound 7A and 7B)

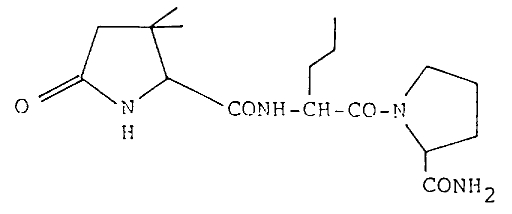

In the same manner as in Example 1, the title less polar compound 7A (215 mg) and the more polar compound 7B (175 mg) were obtained each as a powder from L-norvalyl-L-prolinamide hydrobromide (702 mg) and 3,3-dimethylpyroglutamic acid (250 mg). The compounds 7A and 7B have diastereomeric relationship to each other.
Compound 7A
   NMR (CD₃OD)
   δ ppm: 0.96 (3H, t, J=7.0Hz), 1.02 (3H, s), 1.25 (3H, s), 1.35 - 1.55 (2H, m), 1.55 - 1.75 (1H, m), 1.75 - 1.90 (1H, m), 1.90 - 2.30 (4H, m), 2.04 (1H, d, J=16.5Hz), 2.30 (1H, d, J=16.5Hz), 3.60 - 3.70 (1H, m), 3.83 (1H, s), 3.85 - 3.95 (1H, m), 4.40 (1H, dd, J=8.0Hz and 4.0Hz), 4.64 (1H, dd, J=8.0Hz and 4.0Hz)
   CIMS m/z: 353(M+1)⁺
Compound 7B
   NMR (CD₃OD)
   δ ppm: 0.97 (3H, t, J=7.0Hz), 1.08 (3H, s), 1.26 (3H, s), 1.40 - 1.60 (2H, m), 1.60 - 1.75 (1H, m), 1.75 - 1.90 (1H, m), 1.90 - 2.30 (4H, m), 2.02 (1H, d, J=16.5Hz), 2.35 (1H, d, J=16.5Hz), 3.60 - 3.70 (1H, m), 3.80 (1H, s), 4.41 (1H, dd, J=7.5Hz and 4.0Hz), 4.57 (1H, dd, J=8.0Hz and 4.0Hz)

### Example 8

N-(3,3-Dimethylpyroglutamyl)-L-methionyl-L-prolinamide (Compound 8A and 8B)

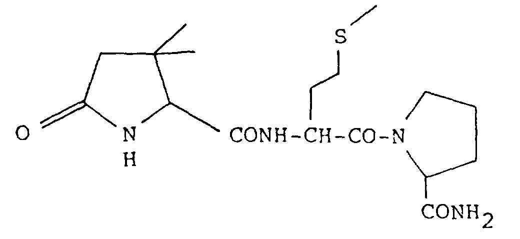

In the same manner as in Example 1, the title less polar compound 8A (212 mg) and the more polar compound 8B (195 mg) were obtained each as a powder from L-methionyl-L-prolinamide hydrobromide (778 mg) and 3,3-dimethylpyroglutamic acid (250 mg). The compounds 8A and 8B have diastereomeric relationship to each other.
Compound 8A
   NMR (CD₃OD)
   δ ppm: 1.03 (3H, s), 1.26 (3H, s), 1.85 - 2.30 (6H, m), 2.04 (1H, d, J=16.5Hz), 2.11 (3H, s), 2.30 (1H, d, J=16.5Hz), 2.50 - 2.70 (2H, m), 3.70 - 3.80 (1H, m), 3.82 (1H, s), 3.90 - 4.00 (lH, m), 4.39 (1H, dd, J=8.0Hz and 4.5Hz)
   CIMS m/z: 385(M+1)⁺
Compound 8B
   NMR (CD₃OD)
   δppm: 1.08 (3H, s), 1.26 (3H, s), 1.85 - 2.30 (6H, m), 2.02 (1H, d, J=16.5Hz), 2.11 (3H, s), 2.36 (1H, d, J=16.5Hz), 2.55 - 2.75 (2H, m), 3.70 - 3.80 (1H, m), 3.79 (1H, s), 3.90 - 4.00 (1H, m), 4.40 (1H, dd, J=8.0Hz and 4.5Hz), 4.81 (1H, dd, J=9.0Hz and 5.0Hz)

### Example 9

### N-(3,3-Dimethylpyroglutamyl)-L-phenylalanyl-L-prolinamide (Compound 9A and 9B)

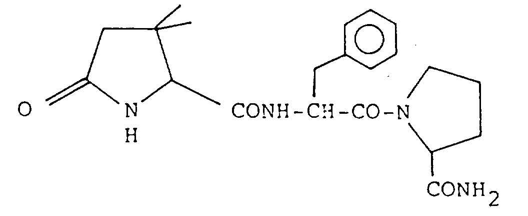

In the same manner as in Example 1, the title less polar compound 9A (206 mg) and the more polar compound 9B (140 mg) were obtained each as a powder from L-phenylalanyl-L-prolinamide hydrobromide (654 mg) and 3,3-dimethylpyroglutamic acid (300 mg). The compounds 9A and 9B have diastereomeric relationship to each other.
Compound 9A
   NMR (CD₃OD)
   δppm: 0.85 (2.3H, s), 1.04 (0.7H, s), 1.21 (2.3H, s), 1.25 (0.7H, s), 1.64 - 2.13 (4H, m), 2.13 - 2.40 (2H, m), 2.91 - 3.15 (1H, m), 3.18 - 3.32 (1H, m), 3.51 - 3.72 (1H, m), 3.74 (0.8H, s), 3.84 (0.2H, s), 3.90 - 4.02 (1H, m), 4.38 - 4.50 (1H, m), 7.20 - 7.42 (5H, m)
   CIMS m/z: 401 (M+1)⁺
Compound 9B
   NMR (CD₃OD)
   δ ppm: 0.61 (2.4H, s), 0.97 (0.6H, s), 1.12 (2.4H, s), 1.23 (0.6H, s), 1.67 - 2.14 (4H, m), 2.14 - 2.42 (2H, m), 2.84 - 3.09 (1H, m), 3.21 - 3.32 (1H, m), 3.61 - 3.72 (1H, m), 3.73 (0.8H, s), 3.80 (0.2H, s), 3.91 - 4.02 (1H, m), 4.38 - 4.50 (1H, m), 7.19 - 7.43 (5H, m)

### Example 10

N-(3,3-Dimethylpyroglutamyl)-L-norleucyl-L-prolinamide (Compound 10A and 10B)

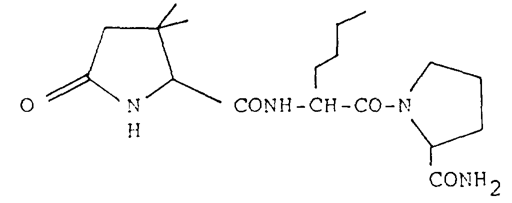

In the same manner as in Example 1, the title less polar compound 10A (175 mg) and the more polar compound 10B (120 mg) were obtained each as a powder from L-norleucyl-L-prolinamide hydrobromide (588 mg) and 3,3-dimethylpyroglutamic acid (300 mg). The compounds 10A and 10B have diastereomeric relationship to each other.
Compound 10A
   NMR (CD₃OD)
   δ ppm: 0.94 (3H, t, J=6.8Hz), 1.03 (3H, s), 1.07 (3H, s), 1.29 - 1.51 (4H, m), 1.58 - 1.76 (1H, m), 1.76 - 2.26 (6H, m), 2.30 (1H, d, J=16.5Hz), 3.59 - 3.71 (1H, m), 3.83 (1H, s), 3.84 - 3.96 (1H, m), 4.39 (1H, dd, J=4.8Hz and 8.2Hz), 4.62 (1H, dd, J=5.2Hz and 9.0Hz)
   CIMS m/z: 367(M+1)⁺
Compound 10B
   NMR (CD₃OD)
   δ ppm: 0.94 (3H, t, J=6.9Hz and 7.1Hz), 1.08 (3H, s), 1.26 (3H, s), 1.30 - 1.51 (4H, m), 1.59 - 1.77 (1H, m), 1.78 - 2.30 (6H, m), 2.36 (1H, d, J=16.4Hz), 3.58 - 3.72 (1H, m), 3.80 (1H, s), 3.81 - 3.93 (1H, m), 4.38 (1H, dd, J=4.7Hz and 8.1Hz), 4.55 (1H, dd, J=4.8Hz and 9.2Hz)

### Example 11

N-[(1R)-3-oxocyclopentanecarbonyl]-L-phenylalanyl-L-prolinamide (Compound 11)

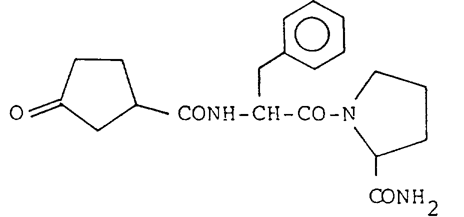

L-Phenylalanyl-L-prolinamide hydrobromide (480 mg) was dissolved in DMF (3 ml), to which was added triethylamine (0.29 ml) under cooling at -10°C. The mixture was stirred for 10 minutes under ice-cooling and the resultant precipitate was filtered off to give L-phenylalanyl-L-prolinamide in DMF solution, which was immediately used for the next reaction.

(1R)-3-Oxocyclopentanecarboxylic acid [K. Toki et al, Bull. Chem. Soc. Jpn.*,* 31, 333 (1958)] (150 mg) was dissolved in DMF (2 ml), to which was added HOBT (216 mg), followed by cooling to 0°C. DCC (265 mg) was added thereto and the mixture was stirred at 0°C overnight. To the mixture was added the above-mentioned L-phenylalanyl-L-prolinamide in DMF, and the mixture was stirred at 5°C overnight. The resultant precipitate was filtered off and concentrated to dryness under reduced pressure. The residue was washed with water and purified by HP-20 column chromatography (1.0x40cm) with methanol as eluent. Further, the eluted portion was concentrated to dryness under reduced pressure, and the residue obtained was purified by silica gel column chromatography (developing solvent/ chloroform:methanol:ammonia water=60:10:1) to give the title compound 11 (150 mg) as powders.
NMR (CD₃OD)
δ ppm: 1.62 - 2.46 (10H, m), 2.81 - 3.27 (3H, m), 3.46 - 3.66 (1H, m), 3.70 - 3.91 (1H, m), 4.40 - 4.51 (1H, m), 7.15 - 7.42 (5H, m)
CIMS m/z: 372(M+1)⁺

### Example 12

N-[(1R)-3-Oxocyclopentanecarbonyl]-L-leucyl-L-prolinamide (Compound 12)

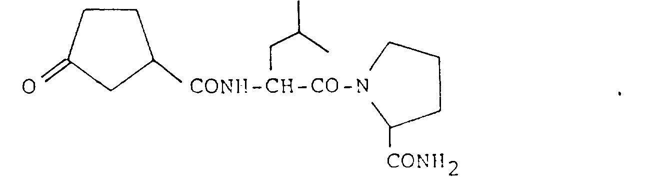

In the same manner as in Example 11, the title compound 12 (280 mg) was obtained as a powder from L-leucyl-L-prolinamide hydrobromide (675 mg) and (1R)-3-oxocyclopentanecarboxylic acid (200 mg).
NMR (CD₃OD)
δ ppm: 0.85 - 1.04 (6H, m), 1.53 - 1.82 (3H, m), 1.85 - 2.45 (10H, m), 3.04 - 3.20 (1H, m), 3.55 - 3.72 (1H, m), 3.76 - 3.92 (1H, m), 4.33 - 4.48 (1H, m), 4.55 - 4.70 (1H, m) CIMS m/z: 338(M+1)⁺

### Example 13

N-(cis-2-Methyl-4-oxocyclopentanecarbonyl)-L-methionyl-L-prolinamide (Compound 13A and 13B)

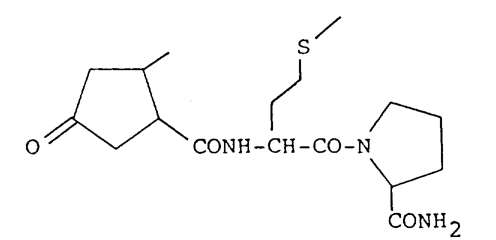

In the same manner as in Example 1, the title less polar compound 13A (152 mg) and a small amount of the more polar compound 13B were obtained as powders from L-methionyl-L-prolinamide hydrobromide (385 mg) and cis-2-methyl-4-oxocyclopentanecarboxylic acid [K. Kojima et al, Chem. Pharm. Bull., 33, 2750 (1985)] (150 mg). The compounds 13A and 13B have diastereomeric relationship to each other. NMR (CD₃OD)
δppm: 1.01 (3H, d, J=6.9Hz), 1.83 - 2.50 (13H, m), 2.50 - 2.74 (3H, m), 3.04 - 3.21 (1H, m), 3.67 - 3.81 (1H, m), 3.83 - 4.02 (1H, m), 4.40 (1H, dd, J=4.6Hz and 8.2Hz), 4.78 (1H, dd, J=5.5Hz and 8.5Hz)
CIMS m/z: 370(M+1)⁺

### Example 14

N-[cis-(1S,2R)-2-Methyl-4-oxocyclopentanecarbonyl]-L-leucyl-L-prolinamide

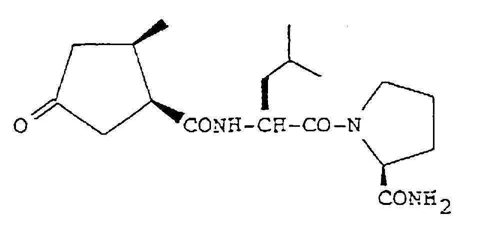

In the same manner as in Example 11, the title compound (342 mg) was obtained as a powder from L-leucyl-L-prolinamide hydrobromide (477 mg) and cis-(1S,2R)-2-methyl-4-oxocyclopentanecarboxylic acid [Haruta et al, Japanese Patent Application No. 188378/1991] (200 mg).
NMR (CD₃OD)
δppm: 0.90 -1.15 (9H, m), 1.55 - 1.80 (3H, m), 1.85 -2.50 (8H, m), 2.55 - 2.70 (1H, m), 3.10 - 3.20 (1H, m), 3.55 - 3.70 (1H, m), 3.85 -3.95 (1H, m), 4.35 - 4.45 (1H, m), 4.60 - 4.70 (1H, m)

### Example 15

N-[cis-(1S,2R)-2-Methyl-4-oxocyclopentanecarbonyl]-L-phenylalanyl-L-prolinamide

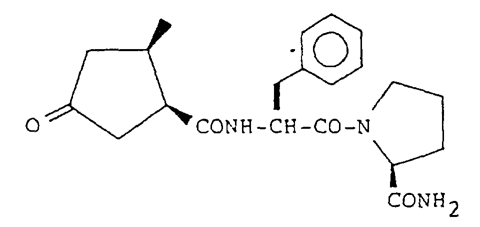

In the same manner as in Example 11, the title compound (450 mg) was obtained as a powder from L-phenylalanyl-L-prolinamide hydrobromide (529 mg) and cis-(1S,2R)-2-methyl-4-oxocyclopentanecarboxylic acid (200 mg).
NMR (CD₃OD)
δppm: 0.99 (2H, d, J=7Hz), 1.09 (2H, d, J=7Hz), 1.60 - 2.50 (8H, m), 2.50 - 2.67 (1H, m), 2.85 - 2.97 (1H, m), 2.97 - 3.10 (1H, m), 3.10 - 3.25 (1H, m), 3.45 - 3.57 (1H, m), 3.80 - 3.93 (1H, m), 4.35 - 4.45 (1H, m), 7.15 - 7.35 (5H, m)

### Example 16

N-[cis-(1S,2R)-2-Methyl-4-oxocyclopentanecarbonyl]-L-norvalyl-L-prolinamide

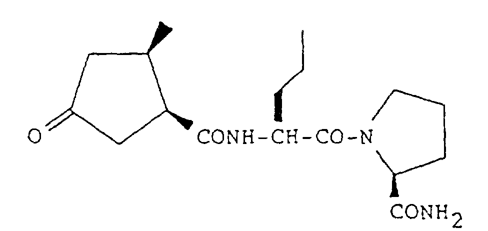

In the same manner as in Example 11, the title compound (345 mg) was obtained as a powder from L-norvalyl-L-prolinamide hydrobromide (400 mg) and cis-(1S,2R)-2-methyl-4-oxocyclopentanecarboxylic acid (200 mg).
NMR (CD₃OD)
δ ppm: 0.90 -1.15 (6H, m), 1.40 - 2.50 (12H, m), 2.55 - 2.70 (1H, m), 3.10 - 3.20 (1H, m), 3.60 - 3.70 (1H, m), 3.85 - 3.95 (1H, m), 4.35 -4.50 (1H, m), 4.50 - 4.65 (1H, m)

### Example 17

N-[cis-(1S,2R)-2-Methyl-4-oxocyclopentanecarbonyl]-L-norleucyl-L-prolinamide

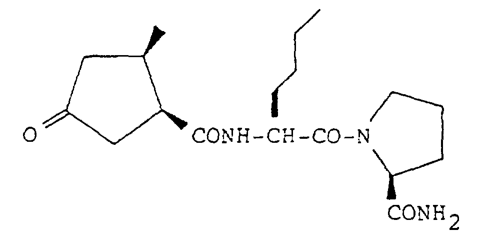

In the same manner as in Example 11, the title compound (227 mg) was obtained as a powder from L-norleucyl-L-prolinamide hydrobromide (477 mg) and cis-(1S,2R)-2-methyl-4-oxocyclopentanecarboxylic acid (200 mg).
NMR (CD₃OD)
δ ppm: 0.90 -1.15 (6H, m), 1.25 - 2.50 (14H, m), 2.56 - 2.70 (1H, m), 3.10 - 3.20 (1H, m), 3.60 - 3.70 (1H, m), 3.85 - 3.95 (1H, m), 4.35 - 4.50 (1H, m), 4.50 - 4.60 (1H, m)

### Example 18

N-(2,2-Dimethyl-4-oxocyclopentanecarbonyl)-L-phenylalanyl-L-prolinamide

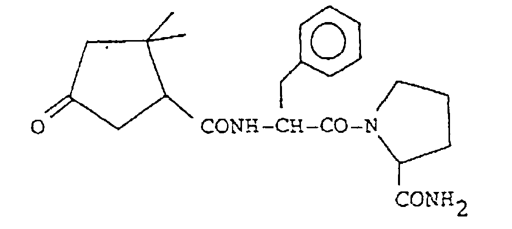

In the same manner as in Example 11, the title less polar compound 18A (38 mg) and the more polar compound 18B (38 mg) were obtained from L-phenylalanyl-L-prolinamide hydrobromide (99 mg) and 2,2-dimethyl-4-oxocyclopentanecarboxylic acid [W. H. Perkin, jun. and J. F. Thorpe, J. Chem. Soc., 79, 729 (1901)] (45 mg). The compounds 18A and 18B have diastereomeric relationship to each other.
Compound 18A
   NMR (CD₃OD)
   δppm: 0.98 (2.4H, s), 1.07 (0.6H, s), 1.18 (2.4H, s), 1.23 (0.6H, s), 1.60 - 2.50 (8H, m), 2.72 - 2.85 (1H, m), 2.85 - 3.10 (1H, m), 3.10 - 3.25 (1H, m), 3.45 -3.55 (1H, m), 3.82 - 3.95 (1H, m), 4.35 - 4.45 (1H, m), 7.15 - 7.40 (5H, m)
Compound 18B
   NMR (CD₃OD)
   δppm: 0.66 (2.4H, s), 0.97 (0.6H, s), 1.00 (2.4H, s), 1.16 (0.6H, s), 1.60 - 2.10 (4H, m), 2.10 - 2.50 (4H, m), 2.72 - 2.80 (1H, m), 2.80 - 2.95 (1H, m), 3.20 - 3.30 (1H, m), 3.60 - 3.70 (1H, m), 3.85 - 3.95 (1H, m), 4.31 - 4.45 (1H, m), 7.15 - 7.40 (5H, m)

### Example 19

N-(2,2-Dimethyl-4-oxocyclopentanecarbonyl)-L-leucyl-L-prolinamide

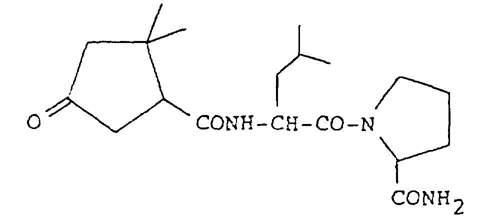

In the same manner as in Example 11, the title less polar compound 19A (50 mg) and the more polar compound 19B (50 mg ) were obtained from L-leucyl-L-prolinamide hydrobromide (99 mg) and 2,2-dimethyl-4-oxocyclopentanecarboxylic acid (45 mg). The compounds 19A and 19B have diastereomeric relationship to each other.
Compound 19A
   NMR (CD₃OD)
   δ ppm: 0.95 (3H, d, J=6.5Hz), 0.98 (3H, d, J=6.5Hz), 1.01 (3H, s), 1.22 (3H, s), 1.55 -1.80 (3H, m), 1.90 -2.40 (6H, m), 2.45 - 2.55 (2H, m), 2.82 - 2.90 (1H, m), 3.57 -3.67 (1H, m), 3.82 - 3.97 (1H, m), 4.35 - 4.45 (1H, m), 4.60 - 4.73 (1H, m)
Compound 19B
   NMR (CD₃OD)
   δ ppm: 0.97 (3H, d, J=6.5Hz), 1.00 (3H, d, J=6.5Hz), 1.07 (3H, s), 1.21 (3H, s), 1.50 -1.70 (2H, m), 1.70 - 1.87 (1H, m), 1.87 -2.30 (5H, m), 2.33 (1H, d, J=18Hz), 2.47 (2H, d, J=6.5Hz), 2.86 (1H, t, J=6.5Hz), 3.55 -3.70 (1H, m), 3.80 - 3.90 (1H, m), 4.35 - 4.45 (1H, m), 4.62 (1H, dd, J=10.5Hz, 4.2Hz)

### Example 20

N-[(1R)-3-Oxocyclopentanecarbonyl]-L-norvalyl-L-prolinamide

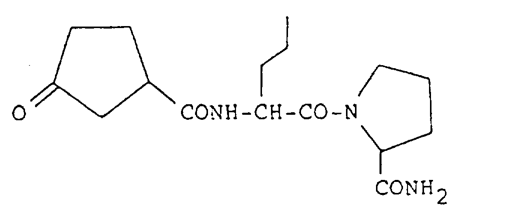

In the same manner as in Example 11, the title compound (322 mg) was obtained as a powder from L-norvalyl-L-prolinamide hydrobromide (379 mg) and (1R)-3-oxocyclopentanecarboxylic acid (150 mg).
NMR (CD₃OD)
δ ppm: 0.96 (3H, t, J=7.2Hz), 1.30 - 1.50 (2H, m), 1.50 - 2.40 (12H, m), 3.05 - 3.20 (1H, m), 3.60 - 3.70 (1H, m), 3.78 - 3.90 (1H, m), 4.41 (1H, dd, J=8.2Hz, 4.6Hz), 4.56 (1H, dd, J=9.1Hz, 4.9Hz)

### Example 21

N-[(1R)-3-Oxocyclopentanecarbonyl]-L-norleucyl-L-prolinamide

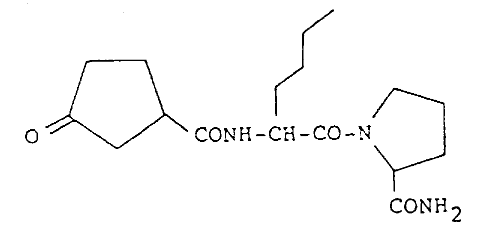

In the same manner as in Example 11, the title compound (243 mg) was obtained as a powder from L-norleucyl-L-prolinamide hydrobromide (397 mg) and (1R)-3-oxocyclopentanecarboxylic acid (150 mg).
NMR (CD₃OD)
δ ppm: 0.94 (3H, t, J=7Hz), 1.25 - 1.50 (4H, m), 1.50 - 2.40 (12H, m), 3.05 - 3.20 (1H, m), 3.57 - 3.70 (1H, m), 3.80 - 3.90 (1H, m), 4.41 (1H, dd, J=8.0Hz, 4.5Hz), 4.54 (1H, dd, J=9.0Hz, 4.8Hz)

Needless to say, the present invention is not limited to these examples, and, for example, the following compounds are also encompassed in the present invention.
22. N-(3-Oxocyclopentanecarbonyl)-L-tyrosyl-L-prolinamide
23. N-(cis-2-Methyl-4-oxocyclopentanecarbonyl)-L-tyrosyl-L-prolinamide
24. N-(2,2-Dimethyl-4-oxocyclopentanecarbonyl)-L-tyrosyl-L-prolinamide
25. N-(cis-2-Methyl-4-oxocyclopentanecarbonyl)-L-isoleucyl-L-prolinanide
26. N-(cis-2-Methyl-4-oxocyclopentanecarbonyl)-L-glutamyl-L-prolinamide
27. N-(cis-2-Methyl-4-oxocyclopentanecarbonyl)-L-asparagyl-L-prolinamide
28. N-(cis-2-Methyl-4-oxocyclopentanecarbonyl)-L-arginyl-L-prolinamide
29. N-(cis-2-Methyl-4-oxocyclopentanecarbonyl)-L-lysyl-L-prolinamide
30. N-(cis-2-Methyl-4-oxocyclopentanecarbonyl)-L-threonyl-L-prolinamide
31. N-(cis-2-Methyl-4-oxocyclopentanecarbonyl)-L-valyl-L-prolinamide
32. N-(cis-2-Ethyl-4-oxocyclopentanecarbonyl)-L-phenylalanyl-L-prolinamide
33. N-(cis-2-Phenyl-4-oxocyclopentanecarbonyl)-L-phenylalanyl-L-prolinamide
34. N-(**3-Oxo**cyclopentanacarbonyl)-L-isoleucyl-L-prolinamide
35. N-(**3-Oxo**cyclopantanecarbonyl)-L-glutamyl-L-prolinamide
36. N-(**3-Oxo**cyclopentanecarbonyl)-L-asparagyl-L-prolinamide
37. N-(**3-Oxo**cyclopentanecarbonyl)-L-arginyl-L-prolinamide
38. N-(**3-Oxo**cyclopentanecarbonyl)-L-lysyl-L-prolinamide
39. N-(**3-Oxo**cyclopentanecarbonyl)-L-seryl-L-prolinamide
40. N-(**3-Oxo**cyclopentanecarbonyl)-L-threonyl-L-prolinamide
41. N-(**3-Oxo**cyclopentanecarbonyl)-L-valyl-L-prolinamide
42. N-(2,2-Dimethyl-4-oxocyclopentanecarbonyl)-L-isoleucyl-L-prolinamide
43. N-(2,2-Dimethyl-4-oxocyclopentanecarbonyl)-L-glutamyl-L-prolinamide
44. N-(2,2-Dimethyl-4-oxocyclopentanecarbonyl)-L-asparagyl-L-prolinamide
45. N-(2,2-Dimethyl-4-oxocyclopentanecarbonyl)-L-arginyl-L-prolinamide
46. N-(2,2-Dimethyl-4-oxocyclopentanecarbonyl)-L-lysyl-L-prolinamide
47. N-(2,2-Dimethyl-4-oxocyclopentanecarbonyl)-L-seryl-L-prolinamide
48. N-(2,2-Dimethyl-4-oxocyclopentanecarbonyl)-L-threonyl-L-prolinamide
49. N-(2,2-Dimethyl-4-oxocyclopentanecarbonyl)-L-valyl-L-prolinamide
50. N-[(cis-2-Methyl-4-oxocyclopentanecarbonyl)-L-phenylalanyl]-L-thiazoline-4-carboxamide
51. N-[(2,2-Dimethyl-4-oxocyclopentanecarbonyl)-L-phenylalanyl]-L-thiazoline-4-carboxamide
52. 3-[N-(3-Methylpyroglutamyl)-L-leucyl]-L-thiazolidine-4-carboxamide
53. 3-[N-(3-Methylpyroglutamyl)-L-methionyl]-L-thiazolidine-4-carboxamide
54. 3-[N-(3-Oxocyclopentanecarbonyl)-L-leucyl]-L-thiazolidine-4-carboxamide
55. 3-[N-(2-Methyl-4-oxocyclopentanecarbonyl)-L-methionyl]-L-thiazolidine-4-carboxamide
56. 3-[N-(2,2-Dimethyl-4-oxocyclopentanecarbonyl)-L-methionyl]-L-thiazolidine-4-carboxamide
57. N-(3-Phenylpyroglutamyl)-L-leucyl-L-prolinamide
58. N-(3-Phenylpyroglutamyl)-L-methionyl-L-prolinamide
59. 3-[N-(3-Phenylpyroqlutamyl)-L-methionyl]-L-thiazolidine-4-carboxamide
60. N-(3-Ethylpyroglutamyl)-L-leucyl-L-prolinamide
61. N-(3-Isopropylpyroglutamyl)-L-methionyl-L-prolinamide
62. 3-[N-(3-Ethylpyroglutamyl)-L-methionyl]-L-thiazolidine-4-carboxamide
63. N-(2-Phenyl-4-oxocyclopentanecarbonyl)-L-methionyl-L-prolinamide
64. N-(3-Methylpyroglutamyl)-L-lysyl-L-prolinamide
65. N-(3-Ethylpyroglutamyl)-L-lysyl-L-prolinamide
66. N-(3-Methylpyroglutamyl)-L-ornithyl-L-prolinamide
67. [(1S)-5-dimethylamino-1-[(2S)-2-carbamoylpyrrolidin-1-ylcarbonyl]propylcarbamoyl]-3-methylpyrrolidin-5-one
68. [(1S)-4-benzyloxycarbonylamino-1-[(2S)-2-carbamoylpyrrolidin-1-ylcarbonyl]butylcarbamoyl]-3-methylpyrrolidin-5-one
69. N-(3-Methylpyroglutamyl)-L-arginyl-L-prolinamide
70. N-(3-Methylpyroglutamyl)-L-threonyl-L-prolinamide
71. [(1S)-2-Methoxy-1-[(2S)-2-carbamoylpyrrolidin-1-ylcarbonyl]propylcarbamoyl]-3-methylpyrrolidin-5-one
72. N-(3-Methylpyroglutamyl)-L-cysteyl-L-prolinamide
73. [(1S)-2-Methylthio-1-[(2S)-2-carbamoylpyrrolidin-1-ylcarbonyl]ethylcarbamoyl]-3-methylpyrrolidin-5-one
74. [(1S)-2-Phenylthio-1-[(2S)-2-carbamoylpyrrolidin-1-ylcarbonyl]ethylcarbamoyl]-3-methylpyrrolidin-5-one
75. [(1S)-2-Methylsulfonyl-1-[(2S)-2-carbamoylpyrrolidin-1-ylcarbonyl]ethylcarbamoyl]-3-methylpyrrolidin-5-one
76. N-(3-Methylpyroglutamyl)-L-asparagyl-L-prolinamide
77. N-(3-Methylpyroglutamyl)-L-tyrosyl-L-prolinamide
78. N-(2-Methyl-4-oxocyclopentanecarbonyl)-L-seryl-L-prolinamide
79. N-(3,3-dimethylpyroglutamyl)-L-arginyl-L-prolinamide
80. [(1S)-4-Acetylamino-1-[(2S)-2-carbamoylpyrrolidin-1-ylcarbonyl]butylcarbamoyl]-3-methylpyrrolidin-5-one
81. [(1S)-2-Acetylamino-1-[(2S)-2-carbamoylpyrrolidin-1-ylcarbonyl]butylcarbamoyl]-3-methylpyrrolidin-5-one

The evaluation tests for anti-reserpine action, anti-chlorpromazine action and spinal reflex stimulant action were conducted as regards the compounds of the present invention.

For comparison, the same tests were conducted also as regards TRH and the hitherto-known DN1417 of the following formula:

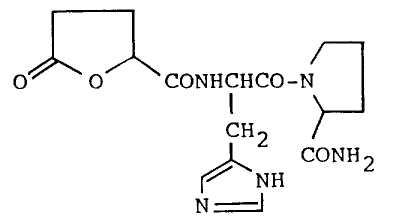

### Test Example 1

### Anti-reserpine action (Antagonistic action against hypothermia)

ICR male mice weighing 35 - 40 g were subcutaneously administered with reserpine at the dose of 5 mg/kg. About 2 hours later, each of the mice was held by a mouse-holder, and a body temperature sensor was intrarectally inserted. Thereafter, the body temperature was automatically measured every 10 minutes. The test compound dissolved in saline was subcutaneously administered 3 or 3.5 hours after administration of reserpine. After the administration of the test compound, the body temperature was measured every 10 minutes over the period of 120 minutes. The area below the body temperature line during the period was calculated, from which the regression line was estimated based upon least square method, and estimated was the amount of the test compound necessary for raising the body temperature by 1°C on the average for 120 minutes from that just before the administration of the test compound (Table 1).

**Table 1**

| | Concentration of test compound necessary for raising body temperature by 1°C on the average for 120 minutes (mg/kg, SC) |
|---|---|
| TRH | 2.52 |
| DN1417 | 2.06 |
| Compound 1A | 0.18 |
| Compound 2A | 0.48 |
| Compound 3A | 0.91 |
| Compound 5A | 0.33 |

### Test Example 2

### Anti-chlorpromazine action (Locomotor stimulant action)

ICR male mice weighing 35 - 40 g (four animals per group) were subcutaneously administered with chlorpromazine HCl at the dose of 5 mg/kg and immediately set in the automatic locomotor-measuring apparatus (AUTOMEX). One hour later, the test compound dissolved in saline was subcutaneously administered, and the locomotor activity was measured every 15 minutes for 120 minutes. The total locomotor activity during the period of 120 minutes was calculated and the regression line was estimated based on the method of least squares, from which the amount of the test compound necessary for inducing 3000 locomotor-count was estimated (Table 2).

**Table 2**

| | Concentration of test compound necessary for inducing 3000 count of automatism quantity in 2 hours (mg/kg, SC) |
|---|---|
| TRH | 6.82 |
| DN1417 | 4.34 |
| Compound 1A | 2.63 |
| Compound 2A | 1.64 |
| Compound 5A | 16.30 |

### Test Example 3

### Flexor reflex in spinalized rats

Wistar male rats (weighing 450 - 520 g) were anesthetized with ether, and the cervical part of spinal cord was exposed, into which a tracheal cannula was inserted. The vagus nerves were severed bilaterally at the cervical region. Thereafter, the cervical vertebra C1 segment was transected, and rapidly, artificial ventilation was given with the use of an artificial respirator (Shinano Factory 60 rpm. 4 ml/stroke). The rat was placed on a thermostated apparatus in which water thermostated at 37°C was circulated and fixed thereto at forelimbs, right hindlimb, tooth and tail. A venous cannula was inserted into the femoral vein in the right hindlimb and two needle electrodes were subcutaneously inserted into the left skin of the toe, and a piece of thread was attached to the limb with the edge of the thread bonded to a force-displacement transducer. Tension of about 5 g was applied to the thread. Through the elctrodes, electric stimuli of 50 - 100 V were applied once per 30 seconds. The flexor reflex was recorded on the polygraph via a force-displacement transducer. After the reaction became constant in about one-hours' lapse for stabilization, the test compound dissolved in saline was intravenously administered. Thereafter, the reaction was recorded for 1 hour. In the data, taking the mean of the three reactions just before administration of the test compound for 100%, the reflex amplitude at 1-, 3-, 5-, 8-, 10-, 15-, 20-, 30-, 40-, 50- and 60- minutes' lapse after administration of the test compound were calculated. In Table 3, there are shown calculated area below the straight line linking the obtained reflex amplitude at the respective above-mentioned time covering 60 minutes in the case of administration of each test compounds at the dose of 0.1 mg/kg, and the reflex amplitude at 10- and 60-minutes' lapse after administration (Table 3).

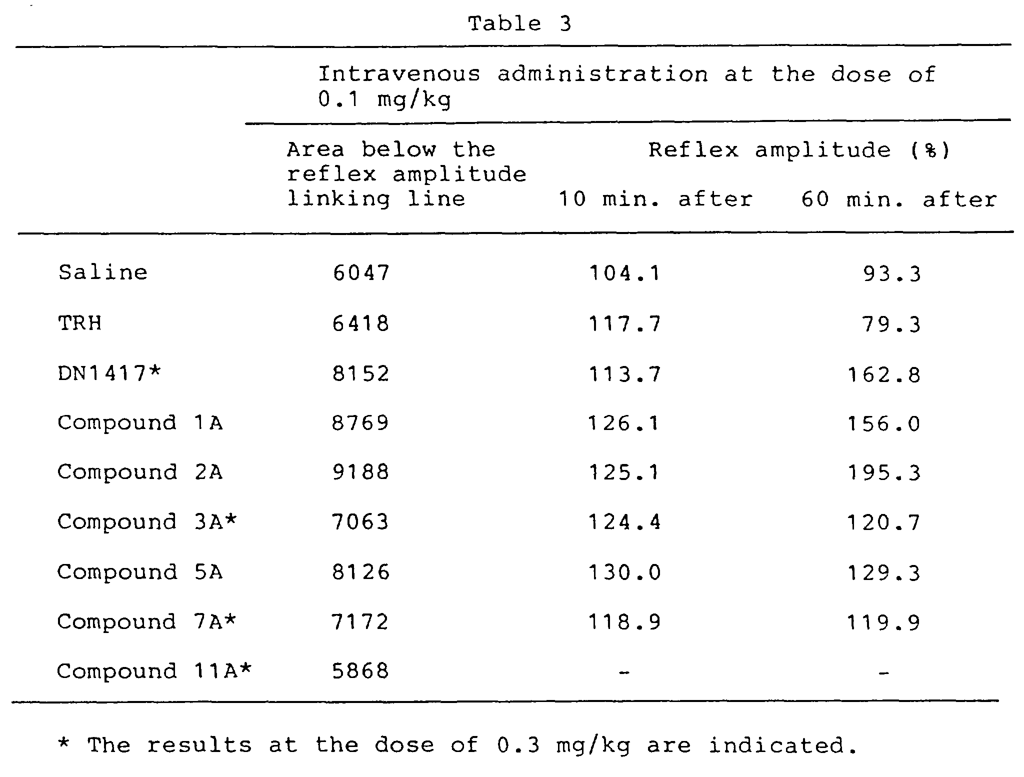

The hormone activity of every compound of the invention was found to be as equal to or less than that of TRH by colloid formation test in thyroid follicles.

## Claims

1. A peptide derivative of the following formula 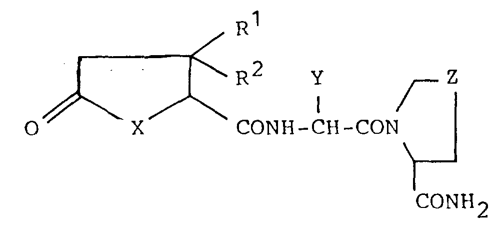 wherein R¹ and R are the same or different and respectively mean a hydrogen atom, a C₁₋₅ alkyl group or a phenyl group, X is -NH- or -CH₂-, Y is a hydrogen atom, a benzyl group wherein phenyl may be substituted by hydroxyl, or a C₁₋₅ alkyl group which may be substituted by -SH, -SR³, -SO₂R³, -CONH₂, 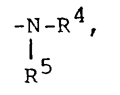 -OR⁶ or 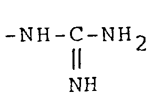 (wherein R³ means a C₁₋₅ alkyl group or an aryl group, R⁴ and R⁵ are the same or different and respectively mean a hydrogen atom, a C₁₋₅ alkyl group or an amino protecting group and R⁶ is a hydrogen atom, a C₁₋₅ alkyl group or a hydroxyl protecting group), and Z is -CH₂- or -S-, provided that when X is -NH-, R¹ and R are not hydrogen atoms at the same time, or a pharmaceutically acceptable acid addition salt thereof.

2. A peptide derivative or a pharmaceutically acceptable acid addition salt thereof as claimed in Claim 1, wherein Y is a C₁₋₅ alkyl which may be substituted by SR³ (wherein R³ is as defined in Claim 1) or a benzyl group.

3. A peptide derivative or a pharmaceutically acceptable acid addition salt thereof as claimed in Claim 1, which is selected from the group consisting of N-(cis-3-methylpyroglutamyl)-L-leucyl-L-prolinamide, N-(cis-3-methylpyroglutamyl)-L-methionyl-L-prolinamide, N-(cis-3-methylpyroglutamyl)-L-norvalyl-L-prolinamide, N-(cis-3-methylpyroglutamyl)-L-phenylalanyl-L-prolinamide, N-(cis-3-methylpyroglutamyl)-L-norleucyl-L-prolinamide, N-(3,3-dimethylpyroglutamyl)-L-leucyl-L-prolinamide, N-(3,3-dimethylpyroglutamyl)-L-norvalyl-L-prolinamide, N-(3,3-dimethylpyroglutamyl)-L-methionyl-L-prolinamide, N-(3,3-dimethylpyroglutamyl)-L-phenylalanyl-L-prolinamide, N-(3,3-dimethylpyroglutamyl)-L-norleucyl-L-prolinamide, N-[(1R)-3-oxocyclopentanecarbonyl]-L-phenylalanyl-L-prolinamide, N-[(1R)-3-oxocyclopentanecarbonyl]-L-leucyl-L-prolinamide, N-(cis-2-methyl-4-oxocyclopentanecarbonyl)-L-methionyl-L-prolinamide, N-[cis-(1S,2R)-2-methyl-4-oxocyclopentanecarbonyl)-L-leucyl-L-prolinamide, N-[cis-(1S,2R)-2-methyl-4-oxocyclopentanecarbonyl]-L-phenylalanyl-L-prolinamide, N-[cis-(1S,2R)-2-methyl-4-oxocyclopentanecarbonyl]-L-norvalyl-L-prolinamide, N-[cis-(1S,2R)-2-methyl-4-oxocyclopentanecarbonyl]-L-norleucyl-L-prolinamide, N-(2,2-dimethyl-4-oxocyclopentanecarbonyl)-L-phenylalanyl-L-prolinamide, N-(2,2-dimethyl-4-oxocyclopentanecarbonyl)-L-leucyl-L-prolinamide, N-[(1R)-3-oxocyclopentanecarbonyl]-L-norvalyl-L-prolinamide and N-[(1R)-3-oxocyclopentanecarbonyl]-L-norleucyl-L-prolinamide.

4. A method of producing a peptide derivative or a pharmaceutically acceptable acid addition salt thereof as claimed in Claim 1, which comprises the steps of
(A) condensing a compound of the formula [III] 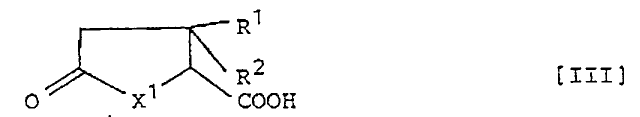 wherein R¹ and R are as defined in Claim 1 and X¹ is an imino group, a protected imino group or a methylene group, or its reactive derivative with a compound of the formula [IV] 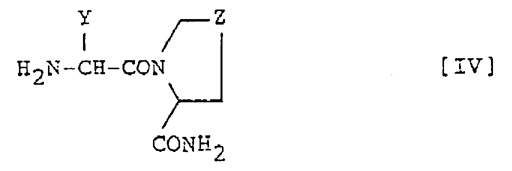 wherein Y and Z are as defined in Claim 1 or its salt;
(B) condensing a compound of the formula [V] 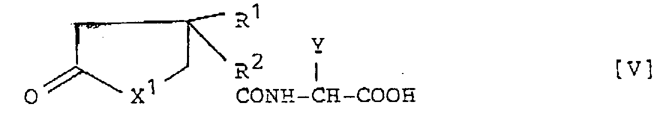 wherein R¹, R, X¹ and Y are as defined above, its salt or its reactive derivative with an amino acid amide of the formula [VI] 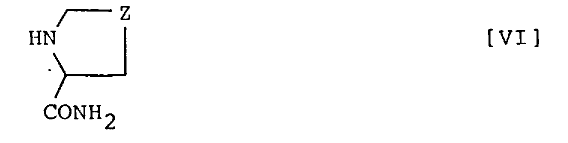 wherein Z is as defined above, or its salt; or
(C) subjecting a compound of the formula [VII] 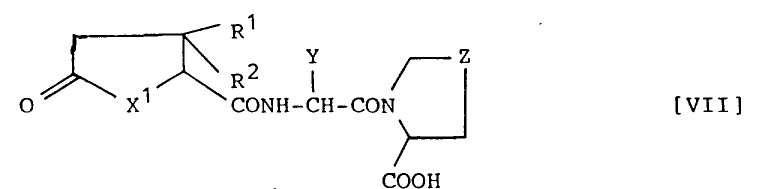 wherein R¹, R, X¹, Y and Z are as defined above, its salt or its reactive derivative to amidation, and
(D) thereafter, when X¹ in the product thus obtained by the above step (A), (B) or (C) is a protected imino group, removing the protective group.

5. A pharmaceutical composition containing a peptide derivatives or a pharmaceutically acceptable acid additon salt thereof as claimed in Claim 1 as an active ingredient.

## Patentansprüche

1. Peptidderivat der folgenden Formel 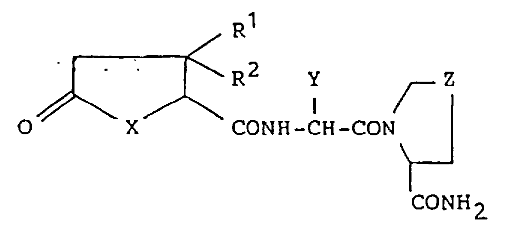 worin R¹ und R gleich oder verschieden sind und jeweils ein Wasserstoffatom, eine C₁₋₅-Alkylgruppe oder eine Phenylgruppe bedeuten, X -NH- oder -CH₂- ist, Y ein Wasserstoffatom, eine Benzylgruppe, in der Phenyl durch Hydroxy substituiert sein kann, oder eine C₁₋₅-Alkylgruppe ist, die durch -SH, -SR³, -SO₂R³, -CONH₂, 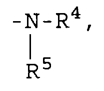 -OR⁶ oder 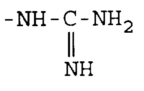 substituiert sein kann (worin R³ eine C₁₋₅-Alkylgruppe oder eine Arylgruppe bedeutet, R⁴ und R⁵ gleich oder verschieden sind und jeweils ein Wasserstoffatom, eine C₁₋₅-Alkylgruppe oder eine Aminoschutzgruppe bedeuten und R⁶ ein Wasserstoffatom, eine C₁₋₅-Alkylgruppe oder eine Hydroxyschutzgruppe ist), und Z -CH₂- oder -S- ist, mit der Maßgabe, daß, wenn X -NH- ist, R¹ und R nicht zugleich Wasserstoffatome sind, oder ein pharmazeutisch annehmbares Säureadditionssalz davon.

2. Peptidderivat oder pharmazeutisch annehmbares Säureadditionssalz davon gemäß Anspruch 1, worin Y eine C₁₋₅-Alkylgruppe, die durch SR³ substituiert sein kann (worin R³ wie in Anspruch 1 definiert ist), oder eine Benzylgruppe ist.

3. Peptidderivat oder pharmazeutisch annehmbares Säureadditionssalz davon gemäß Anspruch 1, das aus der Gruppe ausgewählt wird, bestehend aus N-(cis-3-Methylpyroglutamyl)-L-leucyl-L-prolinamid, N-(cis-3-Methylpyroglutamyl)-L-methionyl-L-prolinamid, N-(cis-3-Methylpyroglutamyl)-L-norvalyl-L-prolinamid, N-(cis-3-Methylpyroglutamyl)-L-phenylalanyl-L-prolinamid, N-(cis-3-Methylpyroglutamyl)-L-norleucyl-L-prolinamid, N-(3,3-Dimethylpyroglutamyl)-L-leucyl-L-prolinamid, N-(3,3-Dimethylpyroglutamyl)-L-norvalyl-L-prolinamid, N-(3,3-Dimethylpyroglutamyl)-L-methionyl-L-prolinamid, N-(3,3-Dimethylpyroglutamyl)-L-phenylalanyl-L-prolinamid, N-(3,3-Dimethylpyroglutamyl)-L-norleucyl-L-prolinamid, N-[(1R)-3-Oxocyclopentancarbonyl]-L-phenylalanyl-L-prolinamid, N-[(1R)-3-Oxocyclopentancarbonyl]-L-leucyl-L-prolinamid, N-(cis-2-Methyl-4-oxocyclopentancarbonyl]-L-methionyl-L-prolinamid, N-[cis-(1S,2R)-2-Methyl-4-oxocyclopentancarbonyl]-L-leucyl-L-prolinamid, N-[cis-(1S,2R)-2-Methyl-4-oxocyclopentancarbonyl]-L-phenylalanyl-L-prolinamid, N-[cis-(1S,2R)-2-Methyl-4-oxocyclopentancarbonyl]-L-norvalyl-L-prolinamid, N-[cis-(1S,2R)-2-Methyl-4-oxocyclopentancarbonyl]-L-norleucyl-L-prolinamid, N-(2,2-Dimethyl-4-oxocyclopentancarbonyl)-L-phenylalanyl-L-prolinamid, N-(2,2-Dimethyl-4-oxocyclopentancarbonyl)-L-leucyl-L-prolinamid, N-[(1R)-3-oxocyclopentancarbonyl]-L-norvalyl-L-prolinamid und N-[(1R)-3-oxocyclopentancarbonyl]-L-norleucyl-L-prolinamid.

4. Verfahren zur Herstellung eines Peptidderivats oder eines pharmazeutisch annehmbaren Säureadditionssalzes davon gemäß Anspruch 1, das die folgenden Schritte umfaßt:
(A) Kondensieren einer Verbindung der Formel (III) 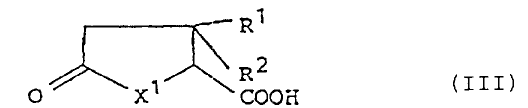 worin R¹ und R wie in Anspruch 1 definiert sind und X¹ eine Iminogruppe, eine geschützte Iminogruppe oder eine Methylengruppe ist, oder ihres reaktiven Derivats mit einer Verbindung der Formel (IV) 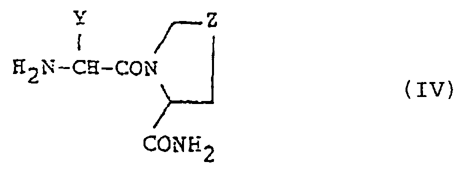 worin Y und Z wie in Anspruch 1 definiert sind, oder ihrem Salz;
(B) Kondensieren einer Verbindung der Formel (V) 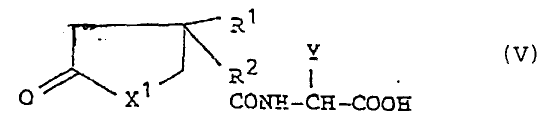 worin R¹, R, X¹ und Y wie oben definiert sind, ihres Salzes oder ihres reaktiven Derivats mit einem Aminosäureamid der Formel (VI) 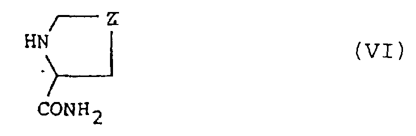 worin Z wie oben definiert ist, oder seinem Salz; oder
(C) Amidieren einer Verbindung der Formel (VII) 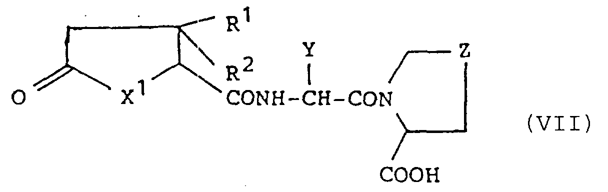 worin R¹, R, X¹, Y und Z wie oben definiert sind, ihres Salzes oder ihres reaktiven Derivats und
(D) danach, wenn X¹ in dem so durch die obigen Schritte (A), (B) oder (C) erhaltenen Produkt eine geschützte Iminogruppe ist, Entfernen der Schutzgruppe.

5. Pharmazeutische Zusammensetzung, die ein Peptidderivat oder ein pharmazeutisch annehmbares Säureadditionssalz davon gemäß Anspruch 1 als aktiven Bestandteil enthält.

## Revendications

1. Un dérivé peptidique de la formule suivante 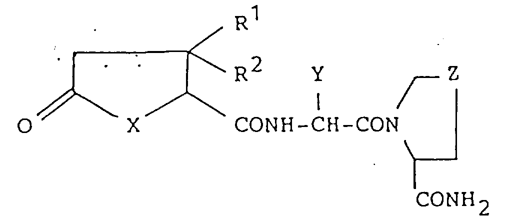 dans laquelle R¹ et R sont identiques ou différents et représentent respectivement, un atome d'hydrogène, un groupe alkyle en C₁ à C₅ ou un groupe phényle, X est -NH- ou -CH₂-, Y est un atome d'hydrogène, un groupe benzyle dans lequel le noyau phényle peut être substitué par de l'hydroxyle ou bien un groupe alkyle en C₁ à C₅ qui peut être substitué par -SH, -SR³, -SO₂R³, -CONH₂, 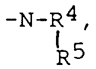 OR⁶ ou 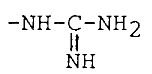 (où R³ signifie un groupe alkyle en C₁ à C₅ ou un groupe aryle, R⁴ et R⁵ sont identiques ou différents et représentent respectivement un atome d'hydrogène, un groupe alkyle en C₁ à C₅ ou un groupe protecteur amino et R⁶ est un atome d'hydrogène, un groupe alkyle en C₁ à C₅ ou un groupe protecteur hydroxyle) et Z est -CH₂- ou -S- sous la condition que lorsque X est -NH-, R¹ et R ne sont pas des atomes d'hydrogène en même temps, ou bien un de ses sels d'addition d'acide, pharmaceutiquement acceptables.

2. Un dérivé peptidique ou un de ses sels d'addition d'acide, pharmaceutiquement acceptable, tel que revendiqué dans la revendication 1, dans lequel Y est un alkyle en C₁ à C₅ qui peut être substitué par SR³ (où R³ étant tel que défini dans la revendication 1) ou un groupe benzyle.

3. Un dérivé peptidique ou un de ses sels d'addition d'acide pharmaceutiquement acceptable tel que revendiqué dans la revendication 1, qui est choisi dans le groupe constitué par les :
N-(cis-3-méthylpyrogluytamyl)-L-leucyl-L-prolinamide, N-(cis-3-méthylpyroglutamyl)-L-méthionyl-L-prolinamide, N-(cis-3-méthylpyroglutamyl)-L-norvalyl-L-prolinamide, N-(cis-3-méthylpyroglutamyl)-L-phénylalanyl-L-prolinamide, N-(cis-3-méthylpyroglutamyl)-L-norleucyl-L-prolinamide, N-(3,3-diméthylpyroglutamyl)-L-leucyl-L-prolinamide, N-(3,3-diméthylpyroglutamyl)-L-norvalyl-L-prolinamide, N-(3,3-diméthylpyroglutamyl)-L-méthionyl-L-prolinamide, N-(3,3-diméthylpyroglutamyl)-L-phénylalanyl-L-prolinamide, N-(3,3-diméthylpyroglutamyl)-L-norleucyl-L-prolinamide, N-[(1R)-3-oxycyclopentanecarbonyl]-L-phénylalanyl-L-prolinamide, N-[(1R)-3-oxyclopentanecarbonyl ]-L-leucyl-L-prolinamide, N- (cis-2-méthyl-4-oxocylopentanecarbonyl-L-méthionyl-L-prolinamide, N-[cis-(1S,2R)-2-méthyl-4-oxocyclopentanecarbonyl)-L-leucyl-L-prolinamide, N-[cis-(1S,2R)-2-méthyl-4-oxocyclopentanecarbonyl]-L-phéylalanlyl-L-prolinamide, N-[cis-(1S,2R)-2-méthyl-4-oxocyclopentanecarbonyl]L-norvalyl-L-prolinamide, N-[cis-(1S,2R)-2-méthyl-4-oxocyclopentanecarbonyl ]-L-norleucyl-L-prolinamide, N-(2,2-diméthyl-4-oxocyclopentanecarbonyl)-L-phénylalanyl-L-prolinamide, N-(2,2-diméthyl-4-oxocyclopentanecarbonyl)-L-leucyl-L-prolinamide, N-[(1R)-3-oxocyclopentanecarbonyl ]-L-norvalyl-L-prolinamide et N-[(1R)-3-oxocyclopentanecarbonyl ]-L-norleucyl-L-prolinamide.

4. Un procédé de préparation d'un dérivé peptidique ou un de ses sels d'addition d'acide, pharmaceutiquement acceptable, tel que revendiqué dans la revendication 1, qui comporte les étapes de
(A) condenser un composé de la formule [III] 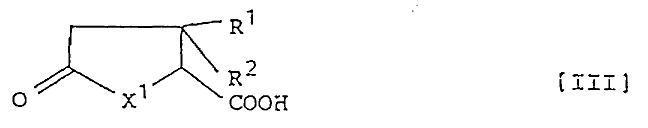 dans laquelle R¹ et R sont tels que définis dans la revendication 1, et X¹ est un groupe imino, un groupe imino protégé ou un groupe méthylène, ou bien son dérivé réactif avec un composé de la formule [IV] 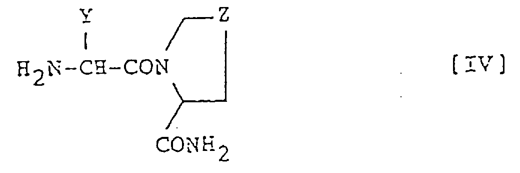 dans laquelle Y et Z sont tels que définis dans la revendication 1 ou son sel;
(B) condenser un composé de la formule [V] 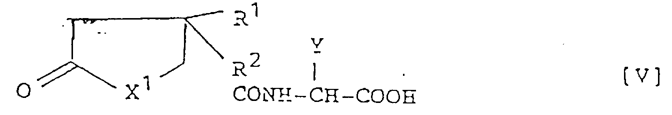 dans laquelle R¹, R, X¹ et Y sont tels que définis ci-dessus, son sel ou son dérivé réactif avec un amide d'acide aminé de la formule [VI] 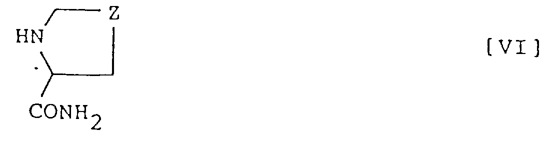 dans laquelle Z est tel que défini ci-dessous, ou son sel; ou
(C) soumettre un composé de la formule [VII] 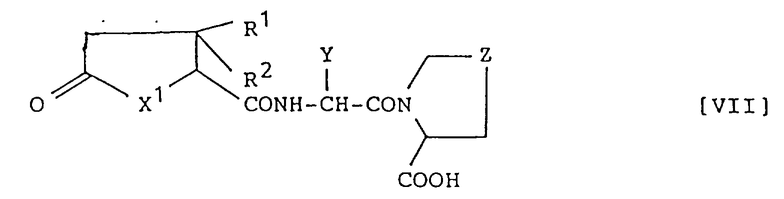 dans laquelle R¹, R, X¹, Y et Z sont tels que définis ci-dessus, son sel ou son dérivé réactif à une amidation, et
(D) par la suite, lorsque X¹ et le produit ainsi obtenu par l'étape (A), (B), ou (C) ci-dessus est un groupe imino protégé, éliminer le groupe protecteur.

5. Une composition pharmaceutique renfermant un dérivé peptidique ou un de ses sels d'addition d'acide, pharmaceutiquement acceptable, tel que revendiqué dans la revendication 1 en tant qu'ingrédient actif.
